Europäisches Patentamt

European Patent Office

Office européen des brevets

(11) Publication number: **0 106 461**
**B1**

## EUROPEAN PATENT SPECIFICATION

(45) Date of publication of patent specification: **10.06.87**

(21) Application number: **83304913.3**

(22) Date of filing: **25.08.83**

(51) Int. Cl.⁴: **A 61 B 10/00**

(54) Gynaecological spatula.

(30) Priority: **14.09.82 GB 8226118**

(43) Date of publication of application:
**25.04.84 Bulletin 84/17**

(45) Publication of the grant of the patent:
**10.06.87 Bulletin 87/24**

(84) Designated Contracting States:
**AT BE CH DE FR GB IT LI LU NL SE**

(56) References cited:
**GB-A-1 429 689**
**US-A-3 583 390**
**US-A-3 774 590**
**US-A-4 016 865**
**US-A-4 318 414**

(73) Proprietor: **Pistofidis, George**
**The Garden Flat 4 Shaftesbury Villas Allen Street**
**London W8 (GB)**

(72) Inventor: **Pistofidis, George**
**The Garden Flat 4 Shaftesbury Villas Allen Street**
**London W8 (GB)**

(74) Representative: **Livsey, Gilbert Charlesworth Norris et al**
**HYDE, HEIDE & O'DONNELL 146 Buckingham Palace Road**
**London SW1W 9TR (GB)**

Courier Press, Leamington Spa, England.

## Description

This invention relates to a gynaecological spatula which may be used for the taking of cervical smears.

The objective of a cervical screening programme is to detect epithelial abnormalities of the cervix at a preinvasive stage. The abnormalities almost always arise at the squamo-columnar junction, extend up into the endocervical canal and down to the ectocervix and vaginal wall.

It is common practice to take cervical smears by a scraping technique, using a spatula. A well-known form of spatula is the Ayer's spatula the distal end of which is generally heart-shaped to provide divergent protuberances with rounded peaks. One of these protuberances is larger than the other and is adapted to enter the cervical opening and function as a pivot when the scraper is rotated. A concave scraping edge extends between that protuberance and the other one and serves to scrape the squamo-columnar junction during such rotation. (See United States Patent 2 471 088). United Kingdom Patent 1 429 689 describes a spatula similar to the Ayer's spatula but wherein the scraping head is of trilobal form, there being a central lobe for entering the cervical opening and two lateral lobes disposed on opposite sides thereof. The lateral lobes both have concave scraping edges and simultaneously scrape the ectocervix. A full 360° scrape of the ectocervix can therefore be obtained without having to turn the spatula to the same extent.

Clinical experience has shown that the endocervical cell content of smears taken from various patient categories by means of prior art spatulas is on average insufficient for the smears to be reliable for the purposes of malignancy detection. There is a positive correlation between the endocervical cell content of smears and the abnormality detection rate.

The detection failures appear largely to be consequential on the many differences among patients, in the size and shape of the cervix and the situation of the squamo-columnar junction. Changes in these features may occur in individual patients from various causes including pregnancy, previous pregnancies, labour, adhesions, tumours or lumps, and previous diagnostic or curative operations on the cervix.

The present invention provides a spatula by means of which cervical smears can be obtained whose endocervical cell content is less dependent on the anatomical factors referred to.

According to the present invention there is provided a gynaecological spatula as defined in claim 1 hereof. A spatula according to the invention has certain features which are known per se in the art, namely the spatula has an elongate body having at its distal end a pair of opposite laterally projecting side lobes with concave leading scraping edges which can bear against the ectocervix and a central projection having side scraping edges which projects forwardly from between said lobes so that it can intrude into the cervical canal while said side lobes are in said bearing position against the ectocervix. However, unlike previously known spatulas, the spatula head is not of fixed shape. Its shape is variable. The elongate body comprises a first component having a distal end portion which forms said central projection, and a second component which carries said laterally projecting side lobes, said first and second components extending to a proximal end portion of the spatula which is held by the user; said first and second components are connected so that said first component is forwardly slidable relative to the second component under manual pressure exerted on said first component at the proximal end portion of the spatula, thereby to lengthen said central projection and cause its tip to advance further along the cervical canal; and said central projection and said side lobes are formed so that at all stages during the said forward sliding movement of said first component, the side scraping edges of said central projection form contiguous extensions of said scraping edges of said side lobes.

The spatula can be inserted along the vagina with the first component (the component providing the said central projection) in retracted position relative to the second component, into a position in which the concave scraping edges of the side lobes bear against the ectocervix and the first component can then be advanced relative to the second component thereby to lengthen the central projection and cause its tip to advance further along the cervical canal. Alternatively the spatula can be inserted along the vagina with the first component in its fully advanced position and this component can be allowed to slide back relative to the second component to some extent depending on the degree of resistance offered by the walls of the cervical canal. Smears are taken by a scraping action, performed by rotating the spatula as a whole. The scraping edges perform a scraping action through 360° on rotation of the spatula through 180°.

Tests have shown that by using a spatula according to the invention a significant improvement in cell abnormality detection rate can be achieved.

The proximal end of the spatula may if desired be formed so that it can be used for scraping the vaginal wall for taking vaginal smears.

In preferred embodiments of the invention the side scraping edges of the central projection provided by the first component converge towards the tip of that projection and the side lobes have tongue portions which bear against those convergent side edges. When the first component is slidden forwardly relatively to the second component, the side lobes therefore become cammed laterally outwards against elastic recovery forces in said second component. In consequence multidirectional movements of the different parts of the spatula head occur.

The scraper components are preferably of flat form. The spatula can be readily and cheaply manufactured from suitable plastics material, i.e. plastics material which can be readily moulded and which is tough.

Examples of plastics which can be used are acrylonitrile/butadiene/styrene polymers and polyolefins such as polyethylene and polypropylene.

Certain embodiments of the invention will hereafter be described by way of example, with reference to the accompanying drawings, in which:

Fig. 1 is a plan view of one spatula according to the invention;

Fig. 2 is a part sectional elevation of that spatula;

Fig. 3 shows the same spatula in use;

Fig. 4 is a transverse cross-section of a spatula body;

Figs. 5 to 7 are views of three differently shaped central scraper portions; and

Figs. 8 and 9 are plan views showing the scraper head of another spatula according to the invention in two different conditions.

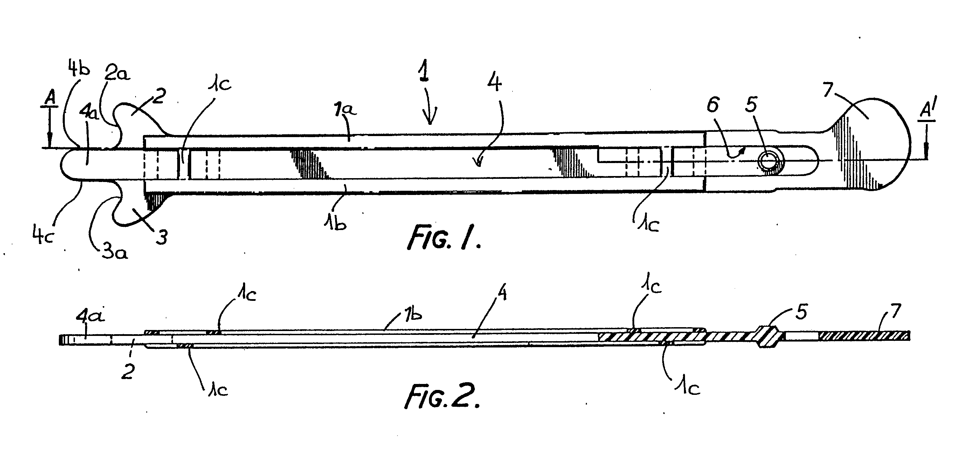

The spatula shown in Figs. 1 and 2 comprises an elongate body formed by components 1 and 4. Component 1 comprises spaced longitudinal side pieces 1a and 1b joined by transverse strips 1c. Component 4 is held between the side pieces 1a, 1b by the strips 1c and is longitudinally slidable relative to the component 1. Component 1 has at one end thereof (called the "distal" end) opposite side lobes 2 and 3 having leading scraping edges 2a, 3a of concave arcuate form. The inner component 4 has a distal end portion 4a which projects centrally between the side lobes 2 and 3. The said projecting end portion 4a has side scraping edges 4b, 4c which are contiguous with and form forward extensions of the concave scraping edges 2a, 3a respectively of the side lobes. By longitudinally sliding component 4 relative to component 1, the distance over which the end portion 4a projects forwardly from the side lobes 2 and 3 can be varied. At the proximal end of component 4 there is a button 5 which projects through a slot 6 in component 1. The button facilitates the manual sliding motion of component 4 relative to component 1.

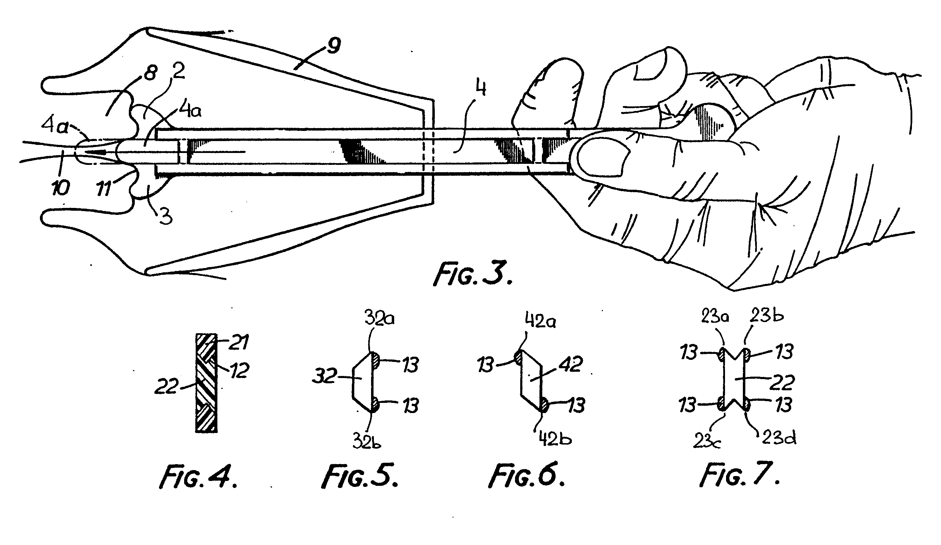

The use of the spatula is illustrated in Fig. 3. A cervix is represented at 8. Vaginal dilation is effected by a conventional speculum 9. The spatula is presented to the cervix with the inner component 4 in its retracted position which is represented in full line. The scraping edges 2a, 3a of the two opposite side lobes 2 and 3 contact the ectocervix, which then follows the concave arcuate profile of these scraping edges as shown at 11. The component 4 is then advanced axially relative to component 1 by exerting forward pressure against the button 5, thereby causing the central scraper portion 4a to advance further along the cervical canal 10 to the position shown in broken line. It will be noted that each side edge of the central scraper portion 4a and the leading concave edge of the corresponding side lobe remain contiguous extensions one of the other so that they together form uninterrupted scraping edges. Rotation of the spatula through 180° then results in scraping of the ectocervix and the adjacent part of the cervical canal through 360°.

The proximal end portion of the component 1 of the spatula shown in Figs. 1 and 2 forms a fixed portion 7 which can be used for taking vaginal smears.

Fig. 4 shows the body portion of another spatula wherein the component 22 carrying the central scraper portion has V-section grooves 12 in its longitudinal edge faces and the other component 21 has edge faces of complementary profile so that the edges of the two components interengage, forming a slide joint.

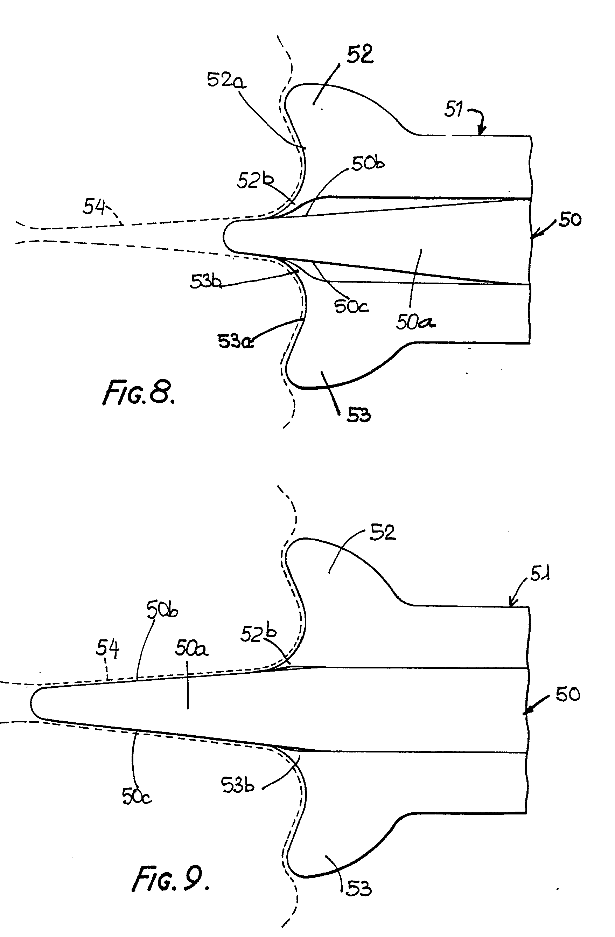

The taking of adequate smears from the cervical canal is promoted if the central projection of the scraping head of a spatula according to the invention is provided with scraping edges which are acutely angled in planes normal to the longitudinal axis of the spatula. Figs. 5 to 7 show different possible transverse cross-sectional shapings of this central projection, which provide such edges. In Fig. 5 the transverse cross-section of the projection 32 is such that it has convergent side edge faces providing two acutely-angled scraping edges 32a, 32b at the ends of its wider face. Rotation of the spatula in one direction produces a build-up of smear 13 on one edge and subsequent rotation in the opposite direction produces a further smear 13 on the other edge. Fig. 6 shows a central projection 42 having parallel chamfered edge faces to provide, as viewed in transverse section, two acutely-angled scraping edges 42a, 42b located at two diagonally opposed corners of the section. With a scraper portion of this section two smears 13 can simultaneously be obtained by turning the spatula in one direction. In Fig. 7 the central projection 23 has V-section grooves along its edge faces so that the projection has four acutely-angled scraping edges 23a—23d by which four smear samples 13 can be obtained from within the cervical canal by turning the spatula first in one direction and then in the other. The V-section grooves can be continued along the component for inter-engagement with guide faces of complementary shape in the other component as has been described with reference to Fig. 4. As an alternative to the V-section grooves, grooves of U-section can be provided.

Referring now to Figs. 8 and 9, these figures show a preferred spatula according to the invention wherein the central projection formed by the distal end portion 50a of a first spatula component 50 is tapered so that its side scraping edges at 50b, 50c converge towards the tip of the projection. The tapering of this distal end portion facilitates its entry into a cervical canal 54, which usually has a progressively diminishing diameter. The second component 51 of the spatula provides opposed side lobes 52, 53 with concave scraping edges 52a, 53a. The side lobes have tongue portions 52b, 53b which bear against the convergent side edges of the tapering distal end portion 50a of the first spatula component 50. As is apparent from a comparison of Figs. 8 and 9, when the first spatula component is slidden forwardly relative to the second component to cause the end portion 50a to advance from its retracted position shown in Fig. 8 to its advanced position shown in Fig. 9, the side lobes 52, 53 are displaced outwardly against elastic recovery forces in the material of the component 51. The tongue portions 52b, 53b elastically press firmly against the side edges 50b, 50c of the tapering central projection 50a and thereby form seals against entry of smear material.

As previously referred to, instead of initially presenting the cervical scraping head of a spatula according to the invention with the central projection in retracted position, the spatula can be presented with the said central projection in fully advanced position and the spatula component carrying this projection can be allowed to slide back relative to the other component to some extent depending on the degree of resistance offered by the walls of the cervical canal.

## Claims

1. A gynaecological spatula for insertion into a cervix and rotation therein for taking cervical smears by a scraping action, said spatula comprising an elongate body having at its distal end a pair of opposite laterally projecting side lobes (2, 3; 52, 53) with concave leading scraping edges (2a, 3a; 52a, 53a) which can bear against the ectocervix and a central projection (4a, 50a) having side scraping edges (4b, 4c; 50b, 50c) which projects forwardly from between said side lobes (2, 3; 52, 53) so that it can intrude into the cervical canal while said side lobes are in said bearing position against the ectocervix, characterised in that:
the elongate body comprises a first component (4; 50) having a distal end portion which forms said central projection (4a; 50a) and a second component (1; 51) which carries said laterally projecting side lobes (2, 3; 52, 53), said first and second components extending to a proximal end portion of the spatula which is held by the user;
said first and second components are connected so that said first component (4; 50) is forwardly slidable relative to the second component (1; 51) under manual pressure exerted on said first component at the proximal end portion of the spatula, thereby to lengthen said central projection (4a; 50a) and cause its tip to advance further along the cervical canal; and
said central projection (4a; 50a) of said first component (4; 50) and said side lobes (2, 3; 52, 53) are formed so that at all stages during the said forward sliding movement of said first component, the side scraping edges (4b, 4c; 50b, 50c) of said central projection form contiguous extensions of said scraping edges (2a, 3a; 52a, 53a) of said side lobes.

2. A spatula according to claim 1, wherein the side scraping edges (50b, 50c) of said central projection (50a) provided by said first component (50) converge towards the tip of that projection and the side lobes (52, 53) carried by the second component (51) have tongue portions (52b, 53b) which bear against those convergent side edges so that such side lobes (52, 53) become cammed laterally outwards against elastic recovery forces in the said second component (51) during said forward sliding movement of the first component.

3. A spatula according to claim 1 or 2, wherein said first component (4; 50) and said second component (1; 51) have longitudinal edge faces (12) of complementary V-profile which interengage, thereby forming a slide joint.

4. A spatula according to any preceding claim, wherein said first component (4; 50) is a flat component having side edge faces shaped so that said central projection (4a; 50a) has side scraping edges (32a, 32b; 42a, 42b; 23a—23d) which are acutely angled in cross-sectional planes normal to the longitudinal axis of the spatula.

5. A spatula according to any preceding claim, wherein at the proximal end portion of said first component (4; 50) it carries an upwardly projecting part (5) against which said manual forward sliding pressure can be exerted.

## Patentansprüche

1. Gynäkologischer Spatel zum Einführen in einen Gebärmutterhals und Verdrehen darin zur Entnahme von Cervikalbelägen durch Schwabwirkung, wobei der Spatel einen langgestreckten Körper umfaßt, mit einem Paar an seinem entfernten Ende gegenüberliegend seitlich vorspringender Seitenlappen (2, 3; 52, 53) mit konkaven vorderseitigen Schabkanten (2a, 3a; 52a, 53a) die gegen die Ectocervix anliegen, und einem mittleren Vorsprung (4a, 50a) mit Seitenschabkanten (4b, 4c; 50b, 50c), der zwischen den Seitenlappen (2, 3; 52, 53) vorwärts vorspringt, so daß er in den Cervikalkanal eindringen kann, während die erwähnten Seitenlappen in der Anlagestellung gegen die Ectocervix sind,
dadurch gekennzeichnet, daß der langgestreckte Körper ein erstes Teil (4; 50) mit einem den mittleren Vorsprung (4a; 50) bildenden, entfernten Endabschnitt und ein zweites Teil (1; 51) umfaßt, das die seitlich vorspringenden Seitenlappen (2, 3; 52, 53) trägt, wobei sich das erste und das zweite Teil zu einem vom Benutzer gehaltenen proximalen Endabschnitt des Spatels erstrecken,
das erste und das zweite Teil so verbunden sind, daß das erste Teil (4; 50) relativ zum zweiten Teil (1; 51) unter einem am proximalen Endabschnitt des Spatels auf das erste Teil ausgeübten manuellen Druck vorwärts verschiebbar ist, um dadurch den mittleren Vorsprung (4a; 50a) zu verlängern und seine Spitze weiter längs des Cervikalkanals vordringen zu lassen, und der Mittelvorsprung (4a; 50a) des ersten Teiles (4; 50) und die Seitenlappen (2, 3; 52, 53) so geformt sind, daß in allen Stadien während der Vortwärtsverschiebung des ersten Teiles die Seitenschabkanten (4b, 4c; 50b, 50c) des mittleren Vorsprungs angrenzende Verlängerungen der Schabkanten (2a, 3a; 52a, 53a) der Seitenlappen bilden.

2. Ein Spatel gemäß Anspruch 1, bei welchem die am ersten Teil (50) vorgesehenen Seitenschabkanten (50b, 50c) des mittleren Vorsprungs (50a) zur Spitze dieses Vorsprunges hin konvergieren und die vom zweiten Teil (51) getragenen Seitenlappen (52, 53) Zungenabschnitte (52b, 53b) besitzen, die gegen diese konvergierenden Seitenkanten anliegen, so daß diese Seitenlappen (52, 53) während der Vorwärtsverschiebung des ersten Teiles gegen elastische Rückstellkräfte im zweiten Teil (51) seitlich auswärts verlagert werden.

3. Ein Spatel gemäß Anspruch 1 oder 2, wobei das erste Teil (4; 50) und das zweite Teil (1; 51) Längskantenflächen (12) von komplementärem V-Profil besitzen, die ineinandergreifen und dadurch eine Schiebefuge bilden.

4. Ein Spatel gemäß jedem der vorhergehenden Ansprüche, wobei das erste Teil (4; 50) ein Flachteil ist mit so geformten Seitenkantenflächen, daß der mittlere Vorsprung (4a; 50a) Seitenschabkanten (32a, 32b; 42a, 42b; 23a—23d) besitzt, die in zur Längsachse des Spatels senkrechten Querschnittsebenen spitzwinklig sind.

5. Ein Spatel gemäß jedem der vorhergehenden Ansprüche, wobei dieser am proximalen Endabschnitt des ersten Teiles (4; 50) ein aufwärts vorspringendes Teil (5) trägt, gegen welches der erwähnte manuelle Vorschubdruck ausgeübt werden kann.

## Revendications

1. Spatule gynécologique destinée à être unsérée dans la cavité cervicale et y être tournée pour prélever un frottis cervical, par action de raclage, ladite spatule comprenant un corps allongé portant à son extrémité distale: deux lobes latéraux (2, 3; 52, 53) qui s'étendent latéralement dans des directions opposées et présentent des bords concaves de raclage (2a, 3a; 52a, 53a) susceptibles de venir porter contre l'ectocervix, et une saillie centrale (4a, 50a) présentant des bords latéraux racleurs (4b, 4c; 50b, 50c) et qui fait saillie vers l'avant à partir de la zone entre les deux lobes latéraux (2, 3; 52, 53) si bien qu'elle peut pénétrer dans le canal cervical alors que lesdits lobes latéraux sont en position d'appui contre l'ectocervix, caractérisée en ce que:

le corps allongé comprend un premier élément (4, 50) comportant une partie d'extrémité distale qui forme ladite saillie centrale (4a, 50a), et un second élément (1, 51) qui porte lesdits lobes faisant saillie latéralement (2, 3; 52, 53), lesdits premier et second éléments s'étendant jusqu'à la partie d'extrémité proximale qui est tenue par l'utilisateur,

lesdits premier et second éléments sont reliés de telle sorte que ledit premier élément (4, 50) peut coulisser vers l'avant par rapport au second élément (1, 51) sous l'effet d'une pression manuelle exercée sur ledit premier élément dans la partie d'extrémité proximale de la spatule, de façon à allonger saillie centrale (4a; 50a) et faire avancer son sommet plus loin dans le canal cervical, et

ladite saillie centrale (4a, 50a) dudit premier élément (4, 50) et lesdits lobes latéraux (2, 3; 52, 53) sont formés de telle sorte que, à toutes les étapes de ce mouvement de coulissement vers l'avant dudit premier élément, les bords latéraux racleurs (4b, 4c; 50b, 50c) de ladite saillie centrale forment des prolongements continus desdits bords de raclage (2a, 3a; 52a, 53a) desdits lobes latéraux.

2. Spatule selon la revendication 1, dans laquelle les bords latéraux racleurs (50b; 50c) de ladite saillie centrale (50a) constituée par ledit premier élément (50) convergent vers le sommet de cette saillie, et les lobes latéraux (52, 53) portés par le second élément (51) comportent des parties en languette (52b, 53b) qui viennent porter contre lesdits bords latéraux qui convergent, si bien que lesdits lobes latéraux (52, 53) sont poussés latéralement vers l'extérieur par effet de came, en opposition avec des formes élastiques de rappel développées par ledit second élément (51), pendant ledit mouvement de coulissement vers l'avant du premier élément.

3. Spatule selon l'une des revendications 1 ou 2, dans laquelle ledit premier élément (4, 50) et ledit second élément (1, 51) ont des faces latérales longitudinales avec des profils en V complémentaires, qui coopèrent pour former un joint coulissant.

4. Spatule selon l'une des revendications précédentes, dans laquelle le premier élément (4, 50) est un élément plat dont les faces des bords latéraux ont une forme telle que ladite saillie centrale (4a, 50a) a des bords latéraux racleurs (32a, 32b; 42a, 42b; 23a à 23d) qui sont à angle aigu dans une section perpendiculaire à l'axe longitudinal de la spatule.

5. Spatule selon l'une des revendications précédentes, dans laquelle ledit premier élément (4, 50) porte, à sa partie d'extrémité proximale, une portion (5) en saillie vers le haut, contre laquelle on peut exercer ladite pression manuelle de coulissement vers l'avant.

FIG. 1.

FIG. 2.

0 106 461

FIG.3.

FIG.4.

FIG.5.

FIG.6.

FIG.7.

0 106 461

*Fig.8.*

*Fig.9.*